(19) Europäisches Patentamt
European Patent Office
Office européen des brevets

(11) Veröffentlichungsnummer: **0 190 563**
**B1**

(12) # EUROPÄISCHE PATENTSCHRIFT

(45) Veröffentlichungstag der Patentschrift: **02.08.89**

(51) Int. Cl.⁴: **C 07 D 471/00**, A 61 K 31/00

(21) Anmeldenummer: **86100255.8**

(22) Anmeldetag: **10.01.86**

(54) **Neue 12-Amino-pyridazino 4',5':3,4 pyrrolo- 2,1-a isochinoline, Verfahren zu ihrer Herstellung und ihre Verwendung.**

(30) Priorität: **14.01.85 DE 3500941**
**13.07.85 DE 3525048**

(43) Veröffentlichungstag der Anmeldung: **13.08.86 Patentblatt 86/33**

(45) Bekanntmachung des Hinweises auf die Patenterteilung: **02.08.89 Patentblatt 89/31**

(84) Benannte Vertragsstaaten:
**AT BE CH DE FR GB IT LI LU NL SE**

(56) Entgegenhaltungen:
**EP-A-0 150 474**
**FR-M-7 348**

(73) Patentinhaber: **BOEHRINGER INGELHEIM KG, Postfach 200, D-6507 Ingelheim am Rhein (DE)**
(84) Benannte Vertragsstaaten:
**BE CH DE FR IT LI LU NL SE AT**

(73) Patentinhaber: **BOEHRINGER INGELHEIM INTERNATIONAL G.M.B.H., Postfach 20, D-6507 Ingelheim am Rhein (DE)**
(84) Benannte Vertragsstaaten: **GB**

(72) Erfinder: **Lösel, Walter, Dr., Im Herzenacker 26, D-6535 Gau- Algesheim (DE)**
Erfinder: **Roos, Otto, Dr., Elsheimer Strasse 36, D-6501 Schwabenheim (DE)**
Erfinder: **Schnorrenberg, Gerd, Dr., Ernst- Ludwig-Strasse 66a, D-6535 Gau- Algesheim (DE)**
Erfinder: **Reichl, Richard, Dr., Im Hippel 55, D-6535 Gau- Algesheim (DE)**
Erfinder: **Ensinger, Helmut, Dr., Im Herrengarten 10, D-6501 Wackernheim (DE)**

**EP 0 190 563 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue 12-Amino-pyridazino[4',5':3,4]-pyrrolo[2,1-a]isochinoline der allgemeinen Formel I

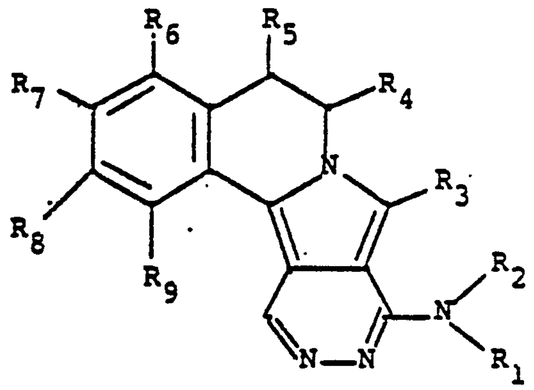

(I)

sowie deren physiologisch verträgliche Säureadditionssalze, Verfahren zu ihrer Herstellung sowie deren Verwendung. In dieser Formel bedeeuten:

$R_1$ und $R_2$, die gleich oder verschieden sein können, Wasserstoff, $C_3$-$C_7$ Cycloalkyl, $C_2$-$C_5$ Alkenyl, Phenyl (wobei der Phenylring gegebenenfalls ein- oder zweifach durch Halogen oder Methoxy substituiert ist), Propargyl, einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Kohlenstoffatomen, Halogen, $NH_2$, NH-Alkyl mit 1 - 2 Kohlenstoffatomen, N,N-Dialkyl mit 1 - 2 Kohlenstoffatomen, NH-Acyl mit 2 - 4 Kohlenstoffatomen, Cycloalkyl mit 3 - 7 Kohlenstoffatomen, Phenyl, (wobei der Phenylring seinerseits weiter substituiert sein kann durch Halogen, Alkyl mit 1 - 2 Kohlenstoffatomen, Alkoxy mit 1 - 2 Kohlenstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen oder Alkylsulfonylamino), Furyl, Thienyl, einen stickstoffhaltigen heterocyclischen 5- oder 6-Ring der gegebenenfalls als weiteres Heteroatom ein Sauerstoff- oder Schwefelatom enthalten kann (wobei der Ring gegebenenfalls durch Alkyl mit 1 - 4 Kohlenstoffatome substituiert ist), $R_1$ und $R_2$ zusammen mit dem Stickstoffatom einen 3- bis 7-gliedrigen Ring, der gegebenenfalls als weiteres Heteroatom ein Sauerstoff- oder ein Stickstoffatom enthalten kann wobei der Ring gegebenenfalls durch Phenyl ($C_1$-$C_4$) Alkyl substituiert ist [wobei der Phenylring seinerseits ein- oder zweifach durch Halogen oder Methoxy substituiert ist)],

$R_2$, falls $R_1$ Wasserstoff bedeutet, einen $-NH_2$, Di($C_1$-$C_2$)Alkylamino, Acetonylamino, -NH($C_2$-$C_3$)Acyl, Alkylsulfonyl- oder Alkoxycarbonylrest mit je 1 - 3 Kohlenstoffatomen in der Alkylkette, die Isopropylidenimi-nogruppe

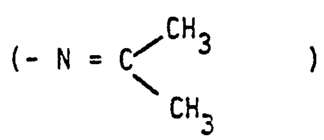

oder einen heterocyclischen, ein Stickstoffatom und gegebenenfalls als weiteres Heteroatom ein Sauerstoff-, Stickstoff- oder Schwefelatom enthaltenden 5- oder 6-Ring,

$R_3$, $R_4$ und $R_5$, die gleich oder verschieden sein können, Wasserstoff oder einen Alkylrest mit 1 - 4 Kohlenstoffatomen,

$R_7$ und $R_8$, die gleich oder verschieden sein können, Hydroxy, Alkoxy mit 1 - 4 Kohlenstoffatomen oder Alkylthio mit 1 - 4 Kohlenstoffatomen und

$R_6$ und $R_9$, die gleich oder verschieden sein können, Wasserstöff, Hydroxy, Alkoxy mit 1 - 4 Kohlenstoffatomen, Alkylthio mit 1 - 4 Kohlenstoffatomen oder den Rest

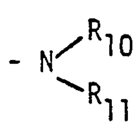

,

in dem

$R_{10}$ Wasserstoff oder Alkyl mit 1 - 4 Kohlenstoffatomen und

$R_{11}$ Wasserstoff oder Alkyl mit 1 - 4 Kohlenstoffatomen, wobei der Alkylrest gegebenenfalls durch Hydroxy, Methoxy oder Furfuryl substituiert sein kann.

Die Erfindung umfaßt ferner die physiologisch verträglichen Säureadditionssalze der Verbindungen der allgemeinen Formel I.

Bevorzugt sind solche Verbindungen der allgemeinen Formel I beziehungsweise deren Säureadditionssalze,

in denen

$R_1$ und $R_2$, die gleich oder verschieden sein können, Wasserstoff oder eine geradkettige oder verzweigte Alkylgruppe mit 1 - 5 Kohlenstoffatomen oder

$R_1$ Wasserstoff und $R_2$ Amino, Methylamino, Dimethylamino, Isopropylidenimino, Dimethylaminoalkyl mit 1 - 4 Kohlenstoffatomen, Methoxyalkyl mit 1 - 4 Kohlenstoffatomen, Cyclopropyl, Cyclopentyl, Cyclohexyl, Cyclohexylmethyl, Phenyl, p-Halogenphenyl, Phenylethyl, wobei der Phenylring gegebenenfalls ein- oder zweifach durch Methoxy substituiert ist, oder Pyrazolyl,

oder $R_1$ und $R_2$ zusammen mit dem Stickstoffatom einen Morpholinoring oder einen Piperazinoring und

$R_7$ und $R_8$ Methoxy bedeuten.

Die neuen Verbindungen können erhalten werden durch Umsetzung eines 3,4-Dihydro-12-methylmercapto-pyridazino-[4',5':3,4]pyrrolo[2,1-a]isochinolins der allgemeinen Formel II

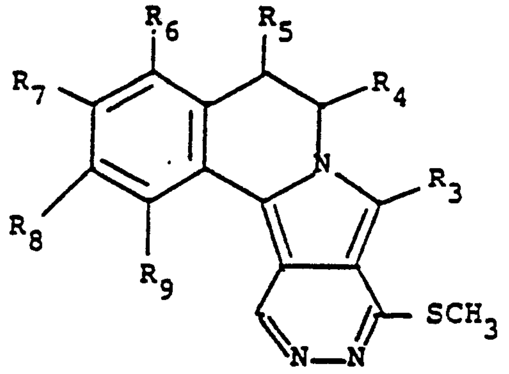

(II)

worin die Reste $R_3$, $R_4$, $R_5$, $R_6$, $R_7$, $R_8$ und $R_9$ die oben angegebene Bedeutung haben, mit einer Verbindung der allgemeinen Formel III

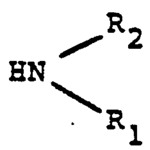

(III)

worin $R_1$ und $R_2$ die oben angegebene Bedeutung haben.

Hierbei wird eine Ausgangsverbindung der allgemeinen Formel II in einem hochsiedenden inerten Lösungsmittel, beispielsweise Dimethylformamid, Dimethylacetamid, Chlorbenzol oder Hexamethylphosphorsäuretrisamid gelöst und mit der Aminkomponente der allgemeinen Formel III unter Rückfluß bis zur Beendigung der Reaktion erhitzt. Die Reaktionszeit beträgt dabei zwischen ca. 1 und 15 Stunden und ist abhängig von den verwendeten Ausgangskomponenten.

Bei reaktionsfähigen Aminen können auch Alkohole oder Tetrahydrofuran als Lösungsmittel verwendet werden; unter Umständen kann auch eine Umsetzung im Autoklav vorteilhaft sein.

Wenn die eingesetzten Amine flüssig und genügend hoch siedend sind, kann die Reaktion auch in einem Überschuß des Amins ohne zusätzliches Lösungsmittel erfolgen (z. B. bei Anilin, Morpholin, Phenylethylamin), gegebenenfalls unter Stickstoffatmosphäre.

In einigen Fällen kann auch ein Reaktionspartner verwendet werden, der bei der Umsetzung sowohl als Lösungsmittel dient als auch durch Spaltung während der Reaktion das benötigte Amin liefert, z. B. Dimethylformamid.

Die erfindungsgemäßen 12-Amino-pyridazino-pyrazolo-isochinoline sind Basen und können auf übliche Weise mit anorganischen oder organischen Säuren in beliebige physiologisch unbedenkliche Säureadditionssalze übergeführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Maleriansäure, Oxalsäure, Malonsäure, Bernsteinsäure, Maleinsäure, Fumarsäure, Milchsäure, Weinsäure, Citronensäure, Apfelsäure, Benzoesäure, p-Hydroxybenzoesäure, p-Aminobenzoesäure, Phthalsäure, Zimtsäure, Salicylsäure, Ascorbinsäure und Methansulfonsäure.

Nach dem oben beschriebenen Verfahren können z. B. die folgenden Endprodukte erhalten werden:

3,4-Dihydro-6,7-dimethoxy-12-N,N-dimethylaminoethyl-amino-pyridazino[4',5':3,4]pyrrolo[2,1-a]isochinolin-hydrochlorid;

3,4-Dihydro-6,7-dimethoxy-12-hydrazino-pyridazino[4',5':3,4]pyrrolo[2,1-a]isochinolin;

3,4-Dihydro-6,7-dimethoxy-12-dimethylamino-pyridazino[4',5':3,4]pyrrolo[2,1-a]isochinolin-hydrochlorid.

3,4-Dihydro-6,7-dimethoxy-12-morpholino-pyridazino[4',5':3,4]pyrrolo[2,1-a]isochinolin-hydrochlorid.

3,4-Dihydro-6,7-dimethoxy-pyridazino[4',5':3,4]pyrrolo[2,1-a]isochinolin-12-acetonylhydrazon.
3,4-Dihydro-6,7-dimethoxy-12-anilino-pyridazino[4',5':3,4)pyrrolo[2,1-a]isochinolin-hydrochlorid.
3,4-Dihydro-6,7-dimethoxy-12-methylamino-pyridazino[4 ,5 :3,4]pyrrolo[2,1-a]isochinolin-hydrochlorid.
3,4-Dihydro-6,7-dimethoxy-12-ethylamino-pyridazino[4 ,5':3,4]pyrrolo[2,1-a]isochinolin-hydrochlorid.
3,4-Dihydro-6,7-dimethoxy-12-diethylamino-pyridazino[4',5':3,4]pyrrolo[2,1-a]isochinolin-hydrochlorid.
3,4-Dihydro-6,7-dimethoxy-12-cyclopropylamino-pyridazino[4',5':3,4]pyrrolo[2,1-a]isochinolin-hydrochlorid.
3,4-Dihydro-6,7-dimethoxy-12-isopropylamino-pyridazino[4',5':3,4]pyrrolo[2,1-a]isochinolin-hydrochlorid.
3,4-Dihydro-6,7-dimethoxy-12-butylamino-pyridazino[4',5 :3,4]pyrrolo[2,1-a]isochinolin-hydrochlorid
3,7-Dihydro-6,7-dimethoxy-12-N,N-dimethylhydrazino-pyridazino[4',5':3,4]pyrrolo[2,1-a]isochinolin-hydrochlorid.
3,4-Dihydro-6,7-dimethoxy-12-(2-methoxyethyl)-aminopyridazino[4',5':3,4]pyrrolo[2,1-a]isochinolin-hydrochlorid.
3,4-Dihydro-6,7-dimethoxy-12-[2-(3,4-dimethoxyphenyl)-ethyl]-amino-pyridazino[4',5':3,4]pyrrolo[2,1-a]isochinolin ·HCl.
3,4-Dihydro-6,7-dimethoxy-12-(3-aminopyrazolyl)-pyridazino[4',5':3,4]pyrrolo[2,1-a]isochinolin-hydrochlorid.
3,4-Dihydro-6,7-dimethoxy-12-cyclopentylamino-pyridazino[4',5:3,4]pyrrolo[2,1-a]isochinolin-hydrochlorid.
3,4-Dihydro-6,7-dimethoxy-12-cyclohexylmethylamino-pyridazino[4',5':3,4]pyrrolo[2,1-a]isochinolin-hydrochlorid.
3,4-Dihydro-6,7-dimethoxy-12-propargylamino-pyridazino[4',5':3,4]pyrrolo[2,1-a]isochinolin-hydrochlorid.
3,4-Dihydro-6,7-dimethoxy-12-{2-[2-(1-methyl)-pyrrolidinyl]-ethyl}-amino-pyridazino[4',5':3,4]pyrrolo[2,1-a]isochinolin ·HCl.
3,4-Dihydro-6,7-dimethoxy-12-[2-(1-piperidinyl)-ethyl]-amino-pyridazino[4',5':3,4]pyrrolo[2,1-a]isochinolin · HCl.
3,4-Dihydro-6,7-dimethoxy-12-pyrrolidinyl-pyridazino[4',5':3,4]pyrrolo[2,1-a]isochinolin-hydrochlorid.
3,4-Dihydro-6,7-dimethoxy-12-n-propylamino-pyridazino[4',5':3,4]pyrrolo[2,1-a]isochinolin-hydrochlorid.
12-Allylamino-3,4-dihydro-6,7-dimethoxy-pyridazino[4',5':3,4]pyrrolo[2,1-a]isochinolin-hydrochlorid.
3,4-Dihydro-6,7-dimethoxy-12-[4-(2-methoxyphenyl)-piperazinyl]-pyridazino[4',5':3,4]pyrrolo[2,1-a]isochinolin-hydrochlorid.
3,4-Dihydro-6,7-dimethoxy-12-(3-dimethylaminopropyl)-amino-pyridazino[4',5':3,4]pyrrolo[2,1-a]isochinolin-hydrochlorid.
3,4-Dihydro-6,7-dimethoxy-12-[2-(1-morpholinyl)-ethyl]-amino-pyridazino[4',5':3,4]pyrrolo[2,1-a]isochinolin · HCl.
3,4-Dihydro-6,7-dimethoxy-12-n-pentylamino-pyridazino[4',5':3 4]pyrrolo[2,1-a]isochinolin-hydrochlorid.
3,4-Dihydro-6,7-dimethoxy-12-cyclohexylamino-pyridazino[4',5':3,4]pyrrolo[2,1-a]isochinolin-hydrochlorid.
3,4-Dihydro-6,7-dimethoxy-12-[4-(2-phenylethyl)-piperazinyl]-pyridazino[4',5':3,4]pyrrolo[2,1-a]isochinolin-hydrochlorid.
3,4-Dihydro-6,7-dimethoxy-12-furfurylamino-pyridazino[4',5':3,4]pyrrolo[2,1-a]isochinolin-hydrochlorid.
3,4-Dihydro-6,7-dimethoxy-12-(3-methoxypropylamino)pyridazino[4',5':3,4]pyrrolo[2,1-a]isochinolin-hydrochlorid.
3,4-Dihydro-6,7-dimethoxy-12-isopentylamino-pyridazino[4',5':3,4]pyrrolo[2,1-a]isochinolin-hydrochlorid.
3,4-Dihydro-6,7-dimethoxy-12-[2-(1-pyrrolidinyl)-ethyl]-amino-pyridazino[4',5':3,4]pyrrolo[2,1-a]isochinolin · HCl.
3,4-Dihydro-6,7-dimethoxy-12-(2-hydroxyethyl)-amino-pyridazino[4',5':3,4]pyrrolo[2,1-a]isochinolin-hydrochlorid.
3,4-Dihydro-6,7-dimethoxy-12-(4-fluoranilino)-pyridazino[4',5':3,4]pyrrolo[2,1-a]isochinolin-hydrochlorid.
3,4-Dihydro-6,7-dimethoxy-12-[2-(4-pyridinyl)-ethyl]-amino-pyridazino[4',5':3,4]pyrrolo[2,1-a]isochinolin-hydrochlorid.
3,4-Dihydro-6,7-dimethoxy-12-[2-(2-pyridinyl)-ethyl]-amino-pyridazino[4',5':3,4]pyrrolo[2,1-a]isochinolin-hydrochlorid.
12-(4-Amino-1-benzyl-piperidinyl)-3,4-dihydro-6,7-dimethoxy-pyridazino[4',5':3,4]pyrrolo[2,1-a]isochinolin · HCl.
3,4-Dihydro-6,7-dimethoxy-12-[2-(3-thieno)-ethyl]-amino-pyridazino[4',5':3,4]pyrrolo[2,1-a]isochinolin-hydrochlorid.
3,4-Dihydro-6,7-dimethoxy-12-{2-[2-(1-methyl)-pyrrolyl]-ethyl}-amino-pyridazino[4',5':3,4]pyrrolo[2,1-a]isochinolin-hydrochlorid.
3,4-Dihydro-6,7-dimethoxy-12-amino-pyridazino[4',5':3,4]pyrrolo[2,1-a]isochinolin-hydrochlorid.
3,4-Dihydro-6,7-dimethoxy-12-[4-(2-phenylethyl)-piperazinyl-]-pyridazino-[4',5 ':3,4]pyrrolo[2,1-a]isochinolin-hydrochlorid.
3,4-Dihydro-6,7-dimethoxy-12-isobutylamino-pyridazino[4',5':3,4]pyrrolo[2,1-a]isochinolin-hydrochlorid.
3,4-Dihydro-6,7-dimethoxy-12-benzylamino-pyridazino[4',5':3,4]pyrrolo[2,1-a]isochinolin-hydrochlorid.

Die Ausgangsstoffe der allgemeinen Formel II können hergestellt werden, ausgehend von einer Verbindung der allgemeinen Formel IV

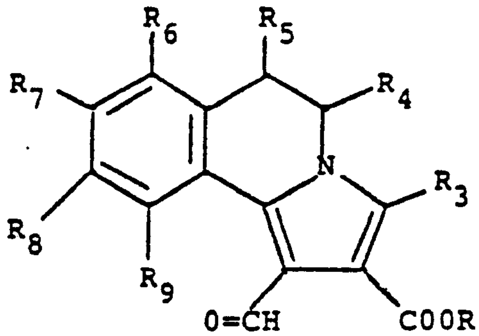

(IV)

worin

R einen niederen Alkylrest mit 1 - 4 Kohlenstoffatomen bedeutet und die Reste $R_3$, $R_4$, $R_5$, $R_6$, $R_7$, $R_8$ und $R_9$ die oben angegebene Bedeutung haben, durch Umsetzung mit Hydrazinhydrat, Umwandlung des so erhaltenen 3,4,11,12-Tetrahydro-pyridazino[4',5':3,4]pyrrolo[2,1-a]isochino-linons-(12) der allgemeinen Formel V

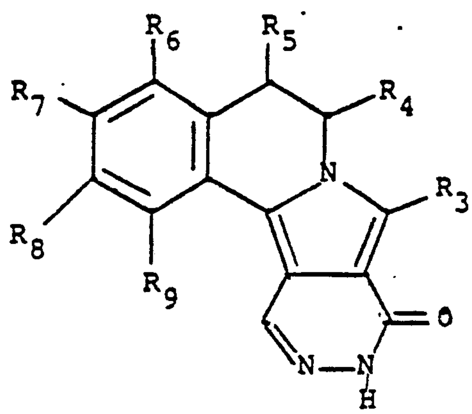

(V)

worin die Reste $R_3$, $R_4$, $R_5$, $R_6$, $R_7$, $R_8$ und $R_9$ die oben angegebene Bedeutung haben,

mit Hilfe von Phosphorpentasulfid in das entsprechende Thion der allgemeinen Formel VI

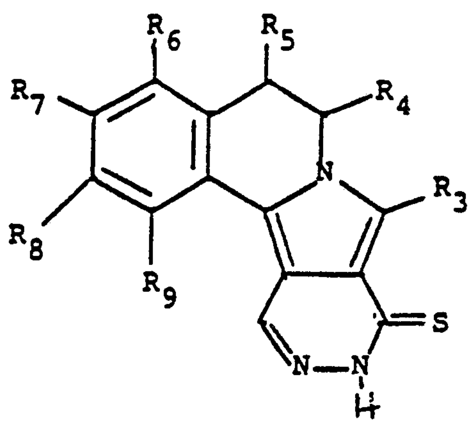

(VI)

worin die Reste $R_3$, $R_4$, $R_5$, $R_6$, $R_7$, $R_8$ und $R_9$ die oben angegebene Bedeutung haben,

und Überführung der Thionverbindung in die entsprechende Methylmercaptoverbindung der allgemeinen Formel II

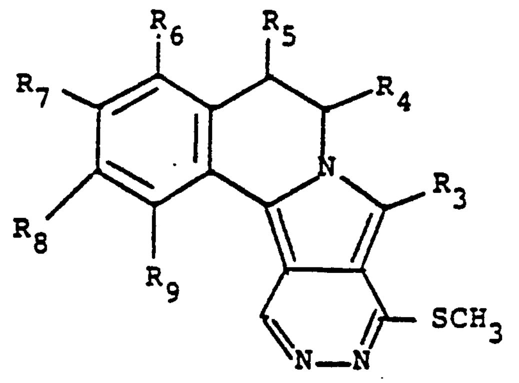

(II)

worin die Reste $R_3$, $R_4$, $R_5$, $R_6$, $R_7$, $R_8$ und $R_9$ die oben angegebene Bedeutung haben mit Hilfe von Dimerhylacetamid/Methyliodid.

Die Ausgangsstoffe der allgemeinen Formel IV können erhalten werden nach den Angaben in der deutschen Patentanmeldung P-3 401 018.1 durch basenkatalysierte Umlagerung der literaturbekannten 1-(3-Furyl)-3,4-dihydroisochinoline.

Die neuen 12-Amino-pyridazino[4',5':3,4]pyrrolo[2,1-a]isochinoline der allgemeinen Formel I besitzen sowohl als Basen als auch in Form ihrer Salze im Tierversuch wertvolle therapeutische Eigenschaften. So zeigen sie eine starke Bindung an den Muscarinrezeptor; dies weist ein Versuch aus, bei dem ein Homogenisat aus verschiedenen Gehirnarealen der Ratte mit Tritium-N-Methylscopolamin inkubiert und durch Zugabe der zu untersuchenden Substanz die Radioliganden verdrängt werden. Die Verdrängung gibt ein Maß für die Affinität der Substanz an den muscarincholinergen Rezeptor. [s. M. Watson et al, Live Science, Bd. 32, S. 3001 - 3011 (1983) und R. Hammer et al, Nature, Bd. 283, S. 90 - 92 (1980)]

Auch in einem Test auf cardiotonische Wirkung am isolierten Herzvorhof des Meerschweinchens haben sich Verbindungen der allgemeinen Formel I als gut wirksam erwiesen. Man verwendet bei diesem Versuch spontanschlagende isolierte Herzvorhöfe; die quantitativ gemessene positiv inotrope Wirkung ist ein Maß für die Kontraktilitätsbeeinflussung. Als $EC_{50}$ wird diejenige Menge an Wirksubstanz in mg/kg bezeichnet, die einen 50 %-igen Anstieg der Kontraktilität bewirkt. (s. R. Reichl, W. Traunecker, A. Engelhardt: Proceedings of the 12th Meeting of the European Society for the Study of Drug Toxicity. Uppsala, June, 1970, Excerpta Medica Int. Congr. Series Nr. 220 - Isolierter Herzvorhof).

Aufgrund dieser Befunde sollen die Verbindungen der allgemeinen Formel I beziehungsweise ihre Säureadditionssalze als Wirkstoffe für Arzneimittel gegen Herzinsuffizienz und cerebraler Stoffwechselstörungen Verwendung finden. Die Verbindungen können sowohl enteral als auch parenteral verabreicht werden. Als Dosis für die orale Anwendung werden 5 bis 500, vorzugsweise 20 bis 250 mg Wirkstoff pro Dosis, für die i. v.-Anwendung 0,5 bis 150, vorzugsweise 5 bis 50 mg pro Dosis vorgeschlagen.

Geeignete Anwendungsformen sind beispielsweise Tabletten, Kapseln, Zäpfchen, Lösungen, Säfte, Emulsionen, Ärosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte mit den erfindungsgemäßen Wirkstoffen bzw. Wirkstoffkombinationen können zusätzlich noch ein Süßungsmittel, wie Saccharin, Cyclamat, Glycerin oder Zucker sowie ein geschmacksverbesserndes Mittel, z. B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z. B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die einen oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln,

wie Neutralfetten oder Polyethylenglykol bzw. dessen Derivaten herstellen.

Die folgenden Beispiele dienen zur näheren Erläuterung der Erfindung:

**Beispiel 1**

3,4-Dihydro-6,7-dimethoxy-12-N,N-dimethylaminoethyl-amino-pyridazino[4',5':3,4]pyrrolo[2,1-a]isochinolin-hydrochlorid

8 g 3,4-Dihydro-6,7-dimethoxy-12-methylmercapto-pyridazino[4',5':3,4]pyrrolo[2,1-a]isochinolin und 15 ml N,N-Dimethylaminoethylamin werden in 100 ml Dimethylacetamid 2 Stunden zum Sieden erhitzt. Nach dem Abkühlen auf Raumtemperatur wird die orangegelbe Lösung im Vakuum eingeengt, der ölige Rückstand in Methylenchlorid aufgenommen, mit Wasser extrahiert und über Natriumsulfat getrocknet. Nach dem Abziehen des Lösungsmittels wird über eine $Al_2O_3$-Säule [$Al_2O_3$ neutral, Fa. Woelm, Aktivitätsstufe III, Eluent: Methylenchlorid/Methanol (100:8)] gereinigt. Das Umsetzungsprodukt wird in Ethanol gelöst und durch Zugabe von ethanolischer Salzsäure als Hydrochlorid gefällt.

Ausbeute: 6,6 g (73 % d. Th.); Fp. > 250° C.

**Beispiel 2**

3,4-Dihydro-6,7-dimethoxy-12-hydrazino-pyridazino[4',5':3,4]pyrrolo[2,1-a]isochinolin

5 g 3,4-Dihydro-6,7-dimethoxy-12-methylmercapto-pyridazino[4',5':3,4]pyrrolo[2,1-a]isochinolin werden in 200 ml Ethanol mit 10 ml Hydrazin-Hydrat 2 Stunden zum Sieden erhitzt. Nach beendeter Umsetzung engt man im Vakuum ein, verrührt den Rückstand mit Wasser, saugt ab und kristallisiert den Filterrückstand aus Ethanol/Ether um. Man erhält die Titelverbindung in einer Ausbeute von 3,4 g (71 % d. Th.); Fp. 254 - 257° C.

**Beispiel 3**

3,4-Dihydro-6,7-dimethoxy-12-dimethylamino-pyridazino[4',5':3,4]pyrrolo[2,1-a]isochinolin-hydrochlorid

5 g 3,4-Dihydro-6,7-dimethoxy-12-methylmercapto-pyridazino[4',5':3,4]pyrrolo[2,1-a]isochinolin werden in 100 ml Dimethylformamid 15 Stunden auf Siedetemperatur erhitzt. Nach beendeter Umsetzung wird eingedampft, der Rückstand in Methylenchlorid aufgeschlämmt und abgesaugt. Nach dem Lösen des Filterrückstandes in heißem Methanol wird die Base durch Zugabe von ethanolischer Salzsäure in das Hydrochlorid überführt und gefällt.

Ausbeute an Titelverbindung: 3,6 g (72 % d. Th.); Fp. 264° C.

Analog Beispiel 3 wurden erhalten:

Ausgehend von 3,4-Dihydro-6,7-dimethoxy-12-methylmercaptopyridazino[4',5':3,4]pyrrolo[2,1-a]isochinolin durch Umsetzung mit Formamid das 3,4-Dihydro-6,7-dimethoxy-12-amino-pyridazino[4',5':3,4]pyrrolo[2,1-a]isochinolinhydrochlorid, Fp. 271° C.

Ausgehend von 3,4-Dihydro-6,7-dimethoxy-12-methylmercapto-pyridazino[4',5':3,4]pyrrolo[2,1-a]isochinolin durch Umsetzung mit Methylformamid das 3,4-Dihydro-6,7-dimethoxy-12-methylamino-pyridazino[4',5':3,4]pyrrolo[2,1-a]isochinolin-hydrochlorid, Fp. 260 - 265° C.

**Beispiel 4**

3,4-Dihydro-6,7-dimethoxy-12-dimethylamino-pyridazino-[4',5':3,4]pyrrolo[2,1-a]isochinolin-hydrochlorid

5 g 3,4-Dihydro-6,7-dimethoxy-12-methylmercapto-pyridazino[4',5':3,4]pyrrolo[2,1-a]isochinolin werden in 50 ml Dimethylacetamid mit 20 ml Dimethylamin 10 Stunden im Autoklav auf 120° C erhitzt. Nach beendeter Umsetzung wird das Lösungsmittel im Vakuum entfernt, der Rückstand an Kieselgel [Eluent: Methylenchlorid /Methanol (100:10)] gereinigt, das Hydrochlorid gebildet und aus Methanol kristallisiert.

Ausbeute: 3,1 g (63 % d. Th.); Fp. 264° C.

Nach der vorstehend beschriebenen Arbeitsweise wurden ferner die folgenden Verbindungen hergestellt:

3,4-Dihydro-6,7-dimethoxy-12-methylamino-pyridazino[4',5':3,4]pyrrolo[2,1-a]isochinolin-hydrochlorid.

Ausbeute: 3,2 g (67 % d. Th.); Fp. > 250° C.

3,4-Dihydro-6,7-dimethoxy-12-ethylamino-pyridazino[4',5':3,4]pyrrolo[2,1-a]isochinolin-hydrochlorid.

Ausbeute: 3,1 g (63 % d. Th.); Fp. 273° C.

3,4-Dihydro-6,7-dimethoxy-12-diethylamino-pyridazino[4',5':3,4]pyrrolo[2,1-a]isochinolin-hydrochlorid,

Ausbeute: 3,4 g (67 % d. Th.); Fp. 276° C.

3,4-Dihydro-6,7-dimethoxy-12-cyclopropylamino-pyridazino[4',5':3,4]pyrrolo[2,1-a]isochinolin-hydrochlorid.

Ausbeute: 2,4 g (46 % d. Th.); Fp. > 260° C.

**Beispiel 5**

3,4-Dihydro-6,7-dimethoxy-12-morpholino-pyridazino[4,5':3,4]pyrrolo[2,1-a]isochinolin-hydrochlorid

5 g 3,4-Dihydro-6,7-dimethoxy-12-methylmercapto-pyridazino[4',5':3,4]pyrrolo[2,1-a]isochinolin und 20 ml Morpholin werden in 100 ml Dimethylacetamid 1,5 Stunden zum Sieden erhitzt. Nach beendeter Umsetzung wird im Vakuum eingeengt, der Rückstand mit Methylenchlorid verrieben und abgesaugt. Das Reaktionsprodukt wird in einem Methylenchlorid/Methanol-Gemisch gelöst, durch Zugabe von ethanolischer Salzsäure in das Hydrochlorid überführt und kristallisiert. Die Ausbeute an Titelverbindung beträgt 3,5 g (63 % d. Th.); Fp. 267° C.

Analog der oben beschriebenen Arbeitsweise wurden hergestellt:

3,4-Dihydro-6,7-dimethoxy-12-isopropylamino-pyridazino[4',5':3,4]pyrrolo[2,1-a]isochinolin-hydrochlorid.
Ausbeute: 3,0 g (57 % d. Th.); Fp. 238° C.

3,4-Dihydro-6,7-dimethoxy-12-butylamino-pyridazino[4',5':3,4]pyrrolo[2,1-a]isochinolin-hydrochlorid.
Ausbeute: 4,2 g (78 % d. Th.); Fp. 257° C.

3,4-Dihydro-6,7-dimethoxy-12-N,N-dimethylhydrazino-pyridazino[4',5':3,4]pyrrolo[2,1-a]isochinolin-hydrochlorid.
Ausbeute: 4,5 g (87 % d. Th.); Fp. > 265° C.

3,4-Dihydro-6,7-dimethoxy-12-(2-methoxyethyl)-aminopyridazino[4',5':3,4]pyrrolo[2,1-a]isochinolin-hydrochlorid.
Ausbeute: 3,6 g (67 % d. Th.); Fp. 252° C.

3,4-Dihydro-6,7-dimethoxy-12-[2-(3,4-dimethoxyphenyl)-ethyl]-amino-pyridazino[4',5':3,4]pyrrolo[2,1-a]isochinolin ·HCl.
Ausbeute: 5,5 g (78 % d. Th)., Fp 257° C.

3,4-Dihydro-6,7-dimethoxy-12-(3-aminopyrazolyl)-pyridazino[4',5':3,4]pyrrolo[2,1-a]isochinolin-hydrochlorid.
Ausbeute: 4,1 g (68 % d. Th.); Fp. > 260° C.

3,4-Dihydro-6,7-dimethoxy-12-cyclopentylamino-pyridazino[4',5':3,4]pyrrolo[2,1-a]isochinolin-hydrochlorid.
Ausbeute: 3,1 g (56 % d. Th.); Fp. 265° C.

3,4-Dihydro-6,7-dimethoxy-12-cyclohexylmethylamino-pyridazino[4',5':3,4]pyrrolo[2,1-a]isochinolin-hydrochlorid.
Ausbeute: 5,2 g (87 % d. Th.); Fp. 262° C.

3,4-Dihydro-6,7-dimethoxy-12-propargylamino-pyridazino[4',5:3,4]pyrrolo[2,1-a]isochinolin-hydrochlorid.
Ausbeute: 3,7 g (73 % d. Th.); Fp. 247° C.

3,4-Dihydro-6,7-dimethoxy-12-{2-[2-(1-methyl)-pyrrolidinyl]-ethyl}-amino-pyridazino[4',5':3,4]pyrrolo[2,1-a]isochinolin ·HCl.
Ausbeute: 4,3 g (69 % d. Th.); Fp. 256 - 258° C.

3,4-Dihydro-6,7-dimethoxy-12-[2-(1-Piperidinyl)-ethyl]-amino-pyridazino[4',5':3,4]pyrrolo[2,1-a]isochinolin · HCl.
Ausbeute: 3,3 g (53 % d. Th.); Fp. 248 - 251° C.

3,4-Dihydro-6,7-dimethoxy-12-pyrrolidinyl-pyridazino[4',5':3,4]pyrrolo[2,1-a]isochinolin-hydrochlorid.
Ausbeute: 3,1 g (59 % d. Th.); Fp. 277 - 278° C.

3,4-Dihydro-6,7-dimethoxy-12-n-propylamino-pyridazino[4',5':3,4]pyrrolo[2,1-a]isochinolin-hydrochlorid.
Ausbeute: 3,7 g ( 72 % d. Th.) ; Fp. 258° C.

12-Allylamino-3,4-dihydro-6,7-dimethoxy-pyridazino[4',5',3,4]pyrrolo[2,1-a]isochinolin-hydrochlorid.
Ausbeute: 2,7 g (53 % d. Th.); Fp. 260 - 263° C.

3,4-Dihydro-6,7-dimethoxy-12-[4-(2-methoxyphenyl)-piperazinyl]-pyridazino[4',5':3,4]pyrrolo[2,1-a]isochinolin-hydrochlorid.
Ausbeute: 4,8 g (66 % d. Th.); Fp. 250 - 252° C.

3,4-Dihydro-6,7-dimethoxy-12-(3-dimethylaminopropyl)-aminopyridazino[4',5':3,4]pyrrolo[2,1-a]isochinolin-hydrochlorid.
Ausbeute: 2,4 g (43 % d. Th.); Fp. > 250° C.

3,4-Dihydro-6,7-dimethoxy-12-[2-(1-morpholinyl)-ethyl]-amino-pyridazino[4',5':3,4]pyrrolo[2,1-a]isochinolin · HCl.
Ausbeute: 3,4 g (54 % d. Th.); Fp. > 260° C.

3,4-Dihydro-6,7-dimethoxy-12-n-pentylamino-pyridazino[4',5 :3,4]pyrrolo[2,1-a]isochinolin-hydrochlorid.
Ausbeute: 4,9 g (87 % d. Th.); Fp. 259 - 261° C.

3,4-Dihydro-6,7-dimethoxy-12-cyclohexylamino-pyridazino[4',5':3,4]pyrrolo[2,1-a]isochinolin-hydrochlorid.
Ausbeute: 3,8 g (66 % d. Th.); Fp. 253° C. .

3,4-Dihydro-6,7-dimethoxy-12-[4-(2-phenylethyl)-piperazinyl]-pyridazino[4',5':3,4]pyrrolo[2,1-a]isochinolin-hydrochlorid.
Ausbeute: 3,8 g (53 % d. Th.); Fp. 264° C.

3,4-Dihydro-6,7-dimethoxy-12-furfurylamino-pyridazino[4',5':3,4]pyrrolo[2,1-a]isochinolin-hydrochlorid.

Ausbeute: 3,2 g (56 % d. Th.); Fp. 243 - 246° C.
3,4-Dihydro-6,7-dimethoxy-12-(3-methoxypropylamino)-pyridazino[4, 5,:3 4]pyrrolo[2 1-a]isochinolin-hydrochlorid.
Ausbeute: 3,6 g (65 % d. Th.); Fp. > 250° C.
3,4-Dihydro-6,7-dimethoxy-12-isopentylamino-pyridazino[4',5':3,4]pyrrolo[2,1-a]isochinolin-hydrochlorid.
Ausbeute: 3,2 g (58 %d. Th.); Fp. 259° C.
3,4-Dihydro-6,7-dimethoxy-12-[2-(1-pyrrolidinyl)-ethyl]-amino-pyridazino[4',5':3,4]pyrrolo[2,1-a]isochinolin HCl.
Ausbeute: 3,3 g (55 % d. Th.); Fp. > 260° C.
3,4-Dihydro-6,7-dimethoxy-12-(2-hydroxyethyl)-amino-pyridazino[4',5':3,4]pyrrolo[2,1-a]isochinolin-hydrochlorid.
Ausbeute: 4,2 g (80 % d. Th.); Fp. 259° C.

**Beispiel 6**

3,4-Dihydro-6,7-dimethoxy-pyridazino[4',5':3,4]pyrrolo[2,1-a]isochinolin-12-acetonylhydrazon
1 g 3,4-Dihydro-6,7-dimethoxy-12-hydrazino-pyridazino[4',5':3,4]pyrrolo[2,1-a]isochinolin werden in 50 ml Aceton gelöst und 1/2 Stunde zum Sieden erhitzt. Man engt im Vakuum ein und saugt die in der Kälte ausfallenden Kristalle ab.
Ausbeute: 5,1 g (95 % d. Th.); Fp. 173 - 175° C.

**Beispiel 7**

3,4-Dihydro-6,7-dimethoxy-12-anilino-pyridazino[4',5':3,4]pyrrolo[2,1-a]isochinolin-hydrochlorid
5 g 3,4-Dihydro-6,7-dimethoxy-12-methylmercapto-pyridazino-[4',5':3,4]pyrrolo[2,1-a]isochinolin werden in 50 ml Anilin unter Stickstoffschutz 6 Stunden zum Sieden erhitzt. Man zieht überschüssiges Anilin im Vakuum ab, verreibt den Rückstand mit Methylenchlorid, saugt ab und kristallisiert aus Methanol/Methylenchlorid um.
Ausbeute: 3,0 g (53 % d. Th.); Fp. > 250° C.
Analog wurden hergestellt:
3,4-Dihydro-6,7-dimethoxy-12-(4-fluoranilino)-pyridazino[4',5':3,4]pyrrolo[2,1-a]isochinolin-hydrochlorid.
Ausbeute: 4,0 g (67 % d. Th.); Fp. > 270° C.
3,4-Dihydro-6,7-dimethoxy-12-[2-(4-pyridinyl)-ethyl]-amino-pyridazino[4',5':3,4]pyrrolo[2,1-a]isochinolin-hydrochlorid.
Ausbeute: 4,1 g (70 % d. Th.); Fp. > 260° C.
3,4-Dihydro-6,7-dimethoxy-12-[2-(2-pyridinyl)-ethyl]-amino-pyridazino[4',5':3,4]pyrrolo[2,1-a]isochinolin-hydrochlorid.
Ausbeute: 3,7 g (62 % d. Th.); Fp. > 260° C.
12-(4-Amino-1-benzyl-piperidinyl)-3,4-dihydro-6,7-dimethoxy-pyridazino[4',5':3,4]pyrrolo[2,1-a]isochinolin HCl.
Ausbeute: 3,9 g (56 % d. Th.); Fp. > 250° C.
3,4-Dihydro-6,7-dimethoxy-12-[2-(3-thieno)-ethyl]-amino-pyridazino[4',5':3,4]pyrrolo[2,1-a]isochinolin-hydrochlorid.
Ausbeute: 4,0 g (65 % d. Th.); Fp. > 280° C.
3,4-Dihydro-6,7-dimethoxy-12-[2-[3-(1-methyl)-pyrrolyl]-ethyl]-amino-pyridazino[4',5':3,4]pyrrolo[2,1-a]isochinolin-hydrochlorid.
Ausbeute: 3,6 g (61 % d. Th.); Fp. 256 - 258° C.
3,4-Dihydro-6,7-dimethoxy-12-[2-(1-piperidinyl)-ethyl]-ammino-pyridazino[4',5':3,4]pyrrolo[2,1-a]isochinolin-hydroyclorid.
Ausbeute: 3,6 g (58 % d. Th.); Fp. 250° C.
3,4-Dihydro-6,7-dimethoxy-12-isobutylamino-pyridazino[4',5':3,4]pyrrolo[2,1-a]isochinolin-hydrochlorid, Fp. 250° C.
3,4-Dihydro-6,7-dimethoxy-12-benzylamino-pyridazino[4',5':3,4]pyrrolo[2,1-a]isochinolin-hydrochlorid, Fp. 257° C.

Die Herstellung der Ausgangsverbindungen wird am Beispiel des 3,4-Dihydro-6,7-dimethoxy-12-methylmercapto-pyridazino[4',5':3,4]pyrrolo[2,1-a]isochinolins beschrieben.

a)3,4,11,12-Tetrahydro-6,7-dimethoxy-pyridazino[4',5:3,4]pyrrolo[2,1-a]isochinolin-12-on
8,25 g 1-Formyl-5,6-dihydro-8,9-dimethoxy-pyrrolo[2,1-a]isochinolin-2-carbonsäureethylester und 7 ml Hydrazin-Hydrat werden 3 Stunden in 40 ml siedendem Pyridin gerührt. Nach dem Erkalten wird der kristalline Niederschlag abgesaugt und durch Waschen mit Methanol von anhaftendem Pyridin befreit. Das anfallende

Produkt ist analysenrein und wird ohne weitere Reinigung für die nächste Stufe eingesetzt.
Ausbeute: 7,1 g (95,4 % d. Th.); Fp. > 285°C.

b) 3,4,11,12-Tetrahydro-6,7-dimethoxy-pyridazino[4',5':3,4]pyrrolo[2,1-a]isochinolin-12-thion

In eine Suspension von 404 g 3,4,11,12-Tetrahydro-6,7-dimethoxy-pyridazino[4',5':3,4]pyrrolo[2,1-a]isochinolin-12-on in 2,8 l Pyridin werden unter Rühren bei Raumtemperatur 302 g Phosphorpentasulfid rasch portionsweise eingetragen. Unter leichtem Temperaturanstieg auf max. 60°C bildet sich eine gelbe Lösung, aus der nach kurzer Zeit orangegelbe Kristalle ausfallen. Man erhitzt das Reaktionsgemisch ca. 4 Stunden unter Rückfluß, läßt auf Raumtemperatur erkalten und saugt das Kristallisat ab. Dieses wird in einem Gemisch aus 1 l Wasser und 200 ml Ethanol digeriert und nach dem Absaugen bei 60°C im Vakuum getrocknet. Das Produkt wird ohne weitere Reinigung weiterverarbeit. Zur Analyse wird eine Probe aus Ethanol/Methylenchlorid kristallisiert.
Ausbeute: 392 g (92 % d. Th.); Fp. 294°C.

c) 3,4-Dihydro-6,7-dimethoxy-12-methylmercapto-pyridazino[4',:3,4]pyrrolo[2,1-a]isochinolin

Eine Suspension von 100 g 3,4,11,12-Tetrahydro-6,7-dimethoxy-pyridazino[4',5':3,4]pyrrolo[2,1-a]isochinolin-12-thion in 600 ml Dimethylacetamid wird bei Raumtemperatur unter Rühren mit 20 ml Methyliodid versetzt. Nach etwa 20 minütigem Rühren bei Raumtemperatur bildet sich eine orangegelbe Lösung, aus der nach weiteren 15 Minuten orangefarbene Kristalle ausfallen. Man läßt eine weitere Stunde rühren, saugt die Kristalle ab und wäscht mit Methylenchlorid nach. Eine zweite Fraktion wird aus der Mutterlauge nach dem Eindampfen und Verreiben mit Methylenchlorid erhalten. Das anfallende Produkt ist NMR-spektroskopisch rein und wird ohne weitere Reinigungsschritte verarbeitet. Eine Probe wurde zur Analyse aus Dimethylacetatamid umkristallisiert.
Ausbeute: 99,8 g (96 % d. Th.); Fp. 254°C.

**Formulierungsbeispiele**

a) Dragees

1 Drageekern enthält:

| | |
|---|---|
| Wirkstoff gemäß der Erfindung | 150,0 mg |
| Milchzucker | 50,0 mg |
| Maisstärke | 22,0 mg |
| Gelatine | 2,0 mg |
| Magnesiumstearat | 1,0 mg |
| | 225,0 mg |

Herstellung:

Die Mischung der Wirksubstanz mit Milchzucker und Maisstärke wird mit einer 10 %-igen wässrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpreßt. Die so erhaltenen Kerne werden in üblicher Weise mit einer Hülle überzogen, die mit Hilfe einer wässrigen Suspension von Zucker, Titandioxid, Talkum und Gummi arabicum aufgebracht wird. Die fertigen Dragees werden mit Bienenwachs poliert.
Dragee-Endgewicht: 200 mg

b) Tabletten

| | |
|---|---|
| Wirkstoff gemäß der Erfindung | 100,0 mg |
| Milchzucker | 40,0 mg |
| Maisstärke | 44,0 mg |
| lösliche Stärke | 5,0 mg |
| Magnesiumstearat | 1,0 mg |
| | 190,0 mg |

Herstellung:

Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 100 mg Gewicht verpreßt, die je 10 mg Wirkstoff enthalten.

c) Suppositorien

1 Zäpfchen enthält:

| | |
|---|---|
| Wirkstoff gemäß der Erfindung | 120,0 mg |
| Zäpfchenmasse | 1680,0 mg |

Herstellung:
Die feingepulverte Substanz wird mit Hilfe eines Eintauch-Homogenisators in die geschmolzene und auf 40° C abgekühlte Zäpfchenmasse eingerührt. Die Masse wird bei 35° C in leicht vorgekühlte Formen gegossen.

d) Ampullen

Zusammensetzung:

| | |
|---|---|
| Wirkstoff gemäß der Erfindung | 50,0 Gew.-Teile |
| Natriumpyrosulfit | 1,0 Gew.-Teile |
| Dinatriumsalz der Ethylendiamin-tetraessigsäure | 0,5 Gew.-Teile |
| Natriumchlorid | 8,5 Gew.-Teile |
| doppelt destilliertes Wasser | ad 1000,0 Gew.-Teile |

Herstellung:
Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullensterilisiert und verschlossen. Jede Ampulle enthält 50,0 mg Wirkstoff.

**Patentansprüche** für die Vertragsstaaten: BE, CH, DE, FR, GB, IT, LI, LU, NL, SE

1. 12-Amino-pyridazino[4',5':3,4]pyrrolo[2,1-a]isochinoline der allgemeinen Formel I

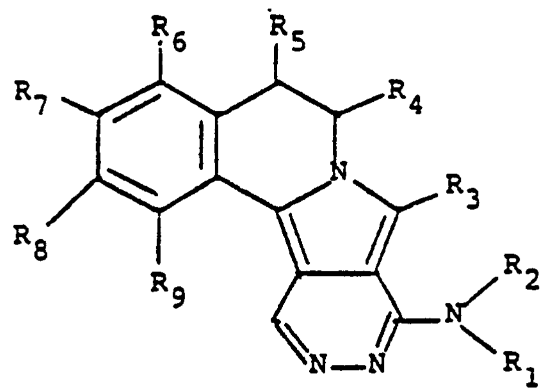

(I)

worin
$R_1$ und $R_2$, die gleich oder verschieden sein können, Wasserstoff, $C_3$-$C_7$ Cycloalkyl, $C_2$-$C_5$ Alkenyl, Phenyl (wobei der Phenylring gegebenenfalls ein- oder zweifach durch Halogen oder Methoxy substituiert ist), Propargyl, einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Kohlenstoffatomen, Halogen, $NH_2$, NH-Alkyl mit 1 - 2 Kohlenstoffatomen, N,N-Dialkyl mit 1 - 2 Kohlenstoffatomen, NH-Acyl mit 2 - 4 Kohlenstoffatomen, Cycloalkyl mit 3 - 7 Kohlenstoffatomen, Phenyl, (wobei der Phenylring seinerseits weiter substituiert sein kann durch Halogen, Alkyl mit 1 - 2 Kohlenstoffatomen, Alkoxy mit 1 - 2 Kohlemstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen, Furyl, Thienyl, einen stickstoffhaltigen heterocyclischen 5- oder 6-Ring, der gegebenenfalls als weiteres Heteroatom ein Sauerstoff- oder Schwefelatom enthalten kann (wobei der Ring gegebenenfalls durch Alkyl mit 1 - 4 Kohlenstoffatome substituiert ist) bedeuten, oder $R_1$ und $R_2$ zusammen mit dem Stickstoffatom einen 3- bis 7-gliedrigen Ring, der gegebenenfalls als weiteres Heteroatom ein Sauerstoff- oder ein Stickstoffatom enthalten kann wobei der Ring gegebenenfalls durch Phenyl ($C_1$-$C_4$) Alkyl substituiert ist (wobei der Phenylring seinerseits ein- oder zweifach durch Halogen oder Methoxy substituiert ist) bedeuten,
oder
$R_2$ falls $R_1$ Wasserstoff bedeutet, einen $-NH_2$, Di($C_1$-$C_2$) Alkylamino, Acetonylamino, -NH($C_2$-$C_3$)Acyl, Alkylsufonyl- oder Alkyloxycarbonylrest mit je 1 - 3 Kohlenstoffatomen in der Alkylkette, die Isopropylideniminogruppe

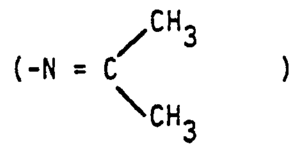

oder einen heterocyclischen, ein Stickstoffatom und gegebenenfalls als weiteres Heteroatom ein Sauerstoff-, Stickstoff- oder Schwefelatom enthaltenden 5- oder 6-Ring bedeutet,

$R_3$, $R_4$ und $R_5$, die gleich oder verschieden sein können, Wasserstoff oder einen Alkylrest mit 1 - 4 Kohlenstoffatomen bedeuten,

$R_7$ und $R_8$, die gleich oder verschieden sein können, Hydroxy, Alkoxy mit 1 - 4 Kohlenstoffatomen oder Alkylthio mit 1 - 4 Kohlenstoffatomen bedeuten, und

$R_6$ und $R_9$, die gleich oder verschieden sein können, für Wasserstoff, Hydroxy, Alkoxy mit 1 - 4 Kohlenstoffatomen, Alkylthio mit 1 - 4 Kohlenstoffatomen oder den Rest

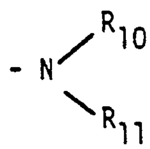

in dem

$R_{10}$ Wasserstoff oder Alkyl mit 1 - 4 Kohlenstoffatomen und $R_{11}$ Alkyl mit 1 - 4 Kohlenstoffatomen, wobei der Alkylrest gegebenenfalls durch Hydroxy, Methoxy oder Furfuryl substituiert sein kann, bedeuten, stehen

und ihre physiologisch verträglichen Säureadditionssalze.

2. Verbindungen der allgemeinen Formel I gemäß Anspruch 1,

worin

$R_1$ und $R_2$, die gleich oder verschieden sein können, Wasserstoff oder eine geradkettige oder verzweigte Alkylgruppe mit 1 - 5 Kohlenstoffatomen oder $R_1$ Wasserstoff und $R_2$ Amino, Methylamino, Dimethylamino, Isopropylidenimino, Dimethylaminoalkyl mit 1 - 4 Kohlenstoffatomen, Methoxyalkyl mit 1 - 4 Kohlenstoffatomen, Cyclopropyl, Cyclopentyl, Cyclohexyl, Cyclohexylmethyl, Phenyl, p-Halogenphenyl, Phenylethyl, wobei der Phenylring gegebenenfalls ein- oder zweifach durch Methoxy substituiert ist, oder Pyrazolyl,

oder $R_1$ und $R_2$ zusammen mit dem Stickstoffatom einen Morpholinoring oder Piperazinoring und

$R_7$ und $R_8$ Methoxy bedeuten,

und ihre physiologisch verträglichen Säureadditionssalze.

3. Die Verbindungen 3,4-Dihydro-6,7-dimethoxy-12-dimethylamino-pyridazino[4',5':3 4]pyrrolo[2,1-a]isochinolin und 3,4-Dihydro-6,7-dimethoxy-12-isopropylamino-pyridazino[4',5':3,4]-pyrrolo[2,1-a]isochinolin

oder ihre Säureadditionssalze.

4. Verfahren zur Herstellung von Verbindungen der allgemeinen Formel I gemäß Anspruch 1 oder ihren Säureadditionssalzen,

dadurch gekennzeichnet, daß man ein 3,4-Dihydro-12-methylmercapto-pyridazino[4',5':3,4)pyrrolo[2,1-a]isochinolin der allgemeinen Formel II

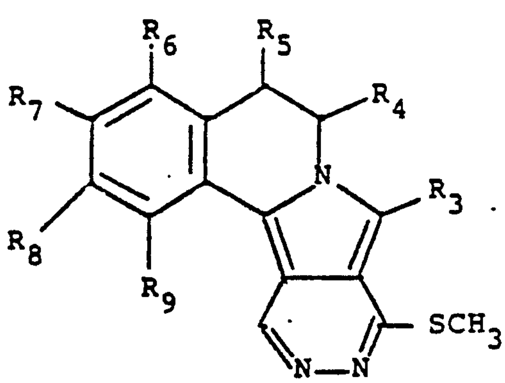

(II)

worin

die Reste $R_3$, $R_4$, $R_5$, $R_6$, $R_7$, $R_8$ und $R_9$ die oben angegebenen Bedeutungen haben, mit einer Verbindung der allgemeinen Formel III

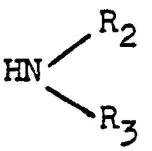

(III)

worin
$R_1$ und $R_2$ die oben angegebene Bedeutung haben, umsetzt, und daß man eine so erhaltene Verbindung gegebenenfalls in an sich bekannter Weise in ein physiologisch verträgliches Säureadditionssalz überführt.

5. Pharmazeutische Präparate, enthaltend als Wirkstoff eine oder mehrere Verbindungen der allgemeinen Formel I in Kombination mit üblichen Hilfs- oder Trägerstoffen.

6. Verfahren zur Herstellung pharmazeutischer Präparate nach Anspruch 5, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

7. Verwendung einer Verbindung nach einem der Ansprüche 1 bis 3 zur Herstellung einer Zubereitung zur Bekämpfung der Herzinsuffizienz und/oder cerebraler Stoffwechselstörungen.

**Patentansprüche** für den Vertragsstaat: AT

1. Verfahren zur Herstellung eines
12-Amino-pyridazino[4',5':3,4]pyrrolo[2,1-a]isochinolins der allgemeinen Formel I

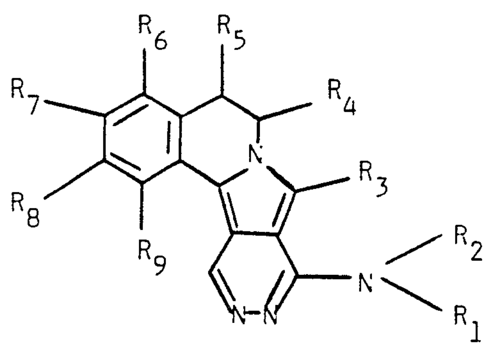

(I)

worin
$R_2$ und $R_2$, die gleich oder verschieden sein können, Wasserstoff, $C_3$-$C_7$ Cycloalkyl, $C_2$-$C_5$ Alkenyl, Phenyl (wobei der Phenylring gegebenenfalls ein- oder zweifach durch Halogen oder Methoxy substituiert ist), Propargyl, einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Konlenstoffatomen, Halogen, $NH_2$, NH-Alkyl mit 1 - 2 Kohlenstoffatomen, N,N-Dialkyl mit 1 - 2 Kohlenstoffatomen, NH-Acyl mit 2 - 4 Kohlenstoffatomen, Cycloalkyl mit 3 - 7 Kohlenstoffatomen, Phenyl (wobei der Phenylring seinerseits weiter substituiert sein kann durch Halogen, Alkyl mit 1 - 2 Kohlenstoffatomen, Alkoxy mit 1 - 2 Kohlenstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen, Furyl, Thienyl, einen stickstoffhaltigen heterocyclischen 5- oder 6-Ring, der gegebenenfalls als weiteres Heteroatom ein Sauerstoff- oder Schwefelatom enthalten kann (wobei der Ring gegebenenfalls durch Alkyl mit 1 - 4 Kohlenstoffatome substituiert ist) bedeuten, oder $R_1$ und $R_2$ zusammen mit dem Stickstoffatom einen 3- bis 7-gliedrigen Ring, der gegebenenfalls als weiteres Heteroatom ein Sauerstoff- oder ein Stickstoffatom enthalten kann [wobei der Ring gegebenenfalls durch Phenyl ($C_1$-$C_4$) Alkyl substituiert ist (wobei der Phenylring seinerseits ein- oder zweifach durch Halogen oder Methoxy substituiert ist)] bedeuten, oder
$R_2$ falls $R_1$ Wasserstoff bedeutet, einen $-NH_2$, Di($C_1$-$C_2$) Alkylamino, Acetonylamino, -NH($C_2$-$C_3$)Acyl, Alkylsulfonyl- oder Alkyloxycarbonylrest mit je 1 - 3 Kohlenstoffatomen in der Alkylkette, die Isopropylideniminogruppe

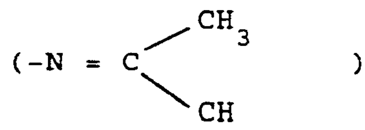

oder einen heterocyclischen, ein Stickstoffatom und gegebenenfalls ale weiteres Heteroatom ein Sauerstoff-, Stickstoff- oder Schwefelatom enthaltenden 5- oder 6-Ring bedeutet, $R_3$, $R_4$ und $R_5$, die gleich oder verschieden sein können, Wasserstoff oder einen Alkylrest mit 1 - 4 Kohlenstoffatomen bedeuten, $R_7$ und $R_8$, die gleich oder verschieden sein können, Hydroxy, Alkoxy mit 1 - 4 Kohlenstoffatomen oder Alkylthio mit 1 - 4 Kohlenstoffatomen bedeuten, und

$R_6$ und $R_9$, die gleich oder verschieden sein können, für Wasserotoff, Hydroxy, Alkoxy mit 1 - 4 Kohlenstoffatomen, Alkylthio mit 1 - 4 Kohlenstoffatomen oder den Rest

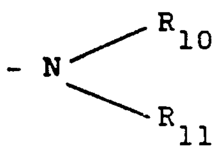

in dem

$R_{10}$ Wasserstoff oder Alkyl mit 1 - 4 Kohlenstoffatomen und $R_{11}$ Alkyl mit 1 - 4 Kohlenstoffatomen, wobei der Alkylrest gegebenenfalls durch Hydroxy, Methoxy oder Furfuryl substituiert sein kann, bedeuten, stehen oder eines physiologisch verträglichen Säureadditionssalzes, dadurch gekennzeichnet, daß man ein 3,4-Dihydro-12-methylmercapto-pyridazino[4',5':3,4]pyrrolo[2,1-a]isochinolin der allgemeinen Formel II

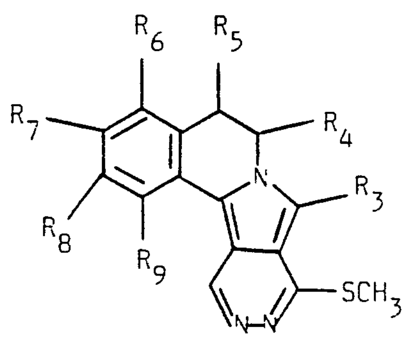

(II)

worin

die Reste $R_3$, $R_4$, $R_5$, $R_6$, $R_7$, $R_8$ und

$R_9$ die oben angegebenen Bedeutungen haben, mit einer Verbindung der allgemeinen Formel III

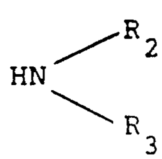

(III)

worin

$R_1$ und $R_2$ die oben angegebene Bedeutung haben,

umsetzt, und daß man eine so erhaltene Verbindung gegebenenfalls in an sich bekannter Weise in ein physiologisch verträgliches Säureadditionssalz überführt.

2. Verfahren nach Anspruch 1, dadurch gekennzeichnet, daß man eine Verbindung der allgemeinen Formel I herstellt, worin

$R_1$ und $R_2$, die gleich oder verschieden sein können, Wasserstoff oder eine geradkettige oder verzweigte Alkylgruppe mit 1 - 5 Kohlenstoffatomen oder $R_1$ Wasserstoff und $R_2$ Amino, Methylamino, Dimethylamino, Isopropylidenimino, Dimethylaminoalkyl mit 1 - 4 Kohlenstoffatomen, Methoxyalkyl mit 1 - 4 Kohlenstoffatomen, Cyclopropyl, Cyclopentyl, Cyclohexyl, Cyclohexylmethyl, Phenyl, p-Halogenphenyl, Phenylethyl, wobei der Phenylring gegebenenfalls ein- oder zweifach durch Methoxy substituiert ist, oder Pyrazolyl,

oder $R_1$ und $R_2$ zusammen mit dem Stickstoffatom einen Morpholinoring oder Piperazinoring und $R_7$ und $R_8$ Methoxy bedeuten, oder ihr physiologisch verträgliches Säureadditionssalz.

3. Verfahren nach Anspruch 1, dadurch gekennzeichnet, daß man 3,4-Dihydro-6,7-dimethoxy-12-dimethylamino-pyridazino[4',5':3,4]pyrrolo[2,1-a]isochinolin oder 3,4-Dihydro-6,7-dimethoxy-12-isopropylamino-pyridazino[4',5':3,4]pyrrolo[2,1-a]isochinolin, oder ihre Säureadditionssalze herstellt.

4. Verwendung einer Verbindung der allgemeinen Formel I oder ihres physiologisch verträglichen Säureadditionssalzes, definiert in einem der Ansprüche 1 bis 3, zur Herstellung einer Zubereitung zur Bekämpfung der Herzinsuffizienz und/oder cerebraler Stoffwechselstörungen.

**Claims** for the Contracting States: BE, CH, DE, FR, GB, IT, LI, LU, NL, SE

1. 12-Amino-pyridazino[4',5':3,4]pyrrolo[2,1-a]isoquinolines of general formula I

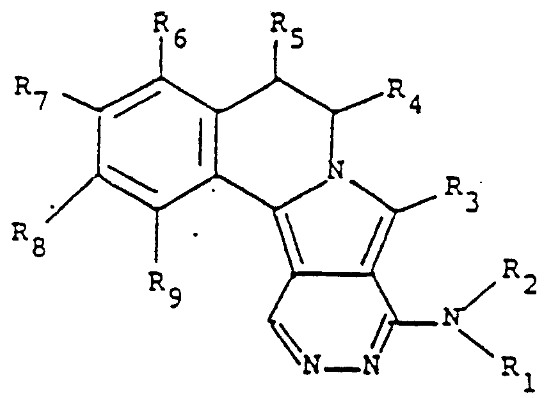

(I)

wherein

$R_1$ and $R_2$, which may be identical or different, each represent hydrogen, $C_{3-7}$ cycloalkyl, $C_{2-5}$ alkenyl, phenyl (optionally mono- or disubstituted by halogen atoms or methoxy groups), propargyl, a straight-chained or branched, saturated or unsaturated alkyl group with 1 to 5 carbon atoms which may be substituted by hydroxy, alkoxy with 1 to 4 carbon atoms, halogen, $NH_2$, NH-alkyl with 1 to 2 carbon atoms, N,N-dialkyl with 1 to 2 carbon atoms, NH-acyl with 2 to 4 carbon atoms, cycloalkyl with 3 to 7 carbon atoms, phenyl (optionally substituted by halogen, alkyl with 1 to 2 carbon atoms, alkoxy with 1 to 2 carbon atoms, NH-alkyl with 1 to 2 carbon atoms, N,N-dialkyl with 1 to 2 carbon atoms, $NH_2$, N-acyl with 2 to 3 carbon atoms), furyl, thienyl, a nitrogen-containing heterocyclic 5- or 6-membered ring which may optionally contain as a further heteroatom an oxygen or sulphur atom (and the ring may optionally be substituted by alkyl with 1 to 4 carbon atoms), or $R_1$ and $R_2$ together with the nitrogen atom represent a 3- to 7-membered ring

which may optionally contain as a further heteroatom an oxygen or nitrogen atom [the ring may be optionally substituted by phenyl ($C_{1-4}$) alkyl (this phenyl ring in turn being mono- or disubstituted by halogen atoms or methoxy groups)],

or $R_2$, if $R_1$ represents hydrogen, represents $-NH_2$, di($C_{1-2}$) alkylamino, acetonylamino, $-NH(C_{2-3})$ acyl, alkylsulphonyl or alkyloxycarbonyl group with 1 to 3 carbon atoms in the alkyl chain, a isopropylideneimino group

(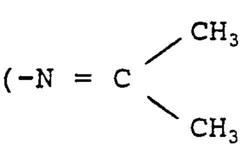)

or a heterocyclic 5- or 6-membered ring containing a nitrogen atom and optionally containing as a further heteroatom an oxygen, nitrogen or sulphur atom,

$R_3$, $R_4$ and $R_5$, which may be identical or different, represent hydrogen or alkyl groups with 1 to 4 carbon atoms,

$R_7$ and $R_8$, which may be identical or different, represent hydroxy, alkoxy with 1 to 4 carbon atoms or alkylthio groups with 1 to 4 carbon atoms, and

$R_6$ and $R_9$, which may be identical or different, represent hydrogen, hydroxy, alkoxy with 1 to 4 carbon atoms or alkylthio groups with 1 to 4 carbon atoms or a group

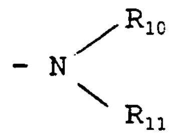

wherein

$R_{10}$ represents hydrogen or alkyl with 1 to 4 carbon atoms and

$R_{11}$ represents alkyl with 1 to 4 carbon atoms any such alkyl group being optionally substituted by a hydroxy, methoxy or furfuryl group

and physiologically acceptable acid addition salts thereof.

2. Compounds of general formula I as claimed in claim 1, wherein

$R_1$ and $R_2$ which may be identical or different represent hydrogen or a straight-chained or branched alkyl group with 1 to 5 carbon atoms or

$R_1$ represents hydrogen and $R_2$ represents amino, methylamino, dimethylamino, isopropylideneimino, dimethylaminoalkyl with 1 to 4 carbon atoms, methoxyalkyl with 1 to 4 carbon atoms, cyclopropyl, cyclopentyl, cyclohexyl, cyclohexylmethyl, phenyl, p-halophenyl, phenylethyl, any such phenyl ring being optionally mono- or disubstituted by methoxy, or pyrazolyl,

or $R_1$ and $R_2$ together with the nitrogen atom to which they are attached represent a morpholino or piperazino ring and

$R_7$ and $R_8$ represent methoxy, and physiologically acceptable acid addition salts thereof.

3. The compounds 3,4-dihydro-6,7-dimethoxy-12-dimethylamino-pyridazino[4',5':3,4]pyrrolo[2,1-a]isoquinoline and 3,4-dihydro-6,7-dimethoxy-12-isopropylamino-pyridazino[4',5':3,4]pyrrolo[2,1-a]isoquinoline or acid addition salts thereof.

4. Process for preparing compounds of general formula I as claimed in claim 1 or the acid addition salts thereof, characterised in that a 3,4-dihydro-12-methyl-mercapto-pyridazino[4',5':3,4]pyrrolo[2,1-a]isoquinoline of general formula II

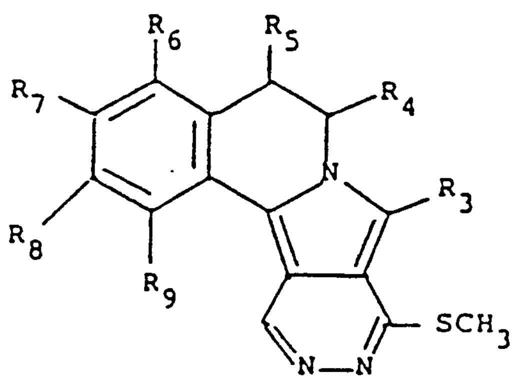

(II)

wherein

the groups $R_3$, $R_4$, $R_5$, $R_6$, $R_7$, $R_8$ and $R_9$ are as hereinbefore defined,

is reacted with a compound of general formula III

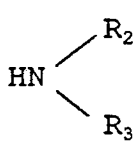

(III)

wherein

$R_1$ and $R_2$ are as hereinbefore defined, and in that a compound thus obtained is optionally converted, in a manner known per se, into a physiologically acceptable acid addition salt.

5. Pharmaceutical preparations containing as active substance one or more compounds of general formula I in conjunction with conventional excipients or carriers.

6. Process for preparing pharmaceutical preparations according to claim 5, characterised in that compounds of general formula I are mixed with conventional galenic excipients and/or carriers to form conventional pharmaceutical preparations.

7. Use of a compound according to any one of claims 1 to 3 for producing the preparation for treating cardiac insufficiency and/or cerebral metabolic disorders.

**Claims** for the Contracting state: AT

1. Process for preparing a 12-amino-pyridazino[4',5':3,4]pyrrolo[2,1-a]isoquinoline of general formula

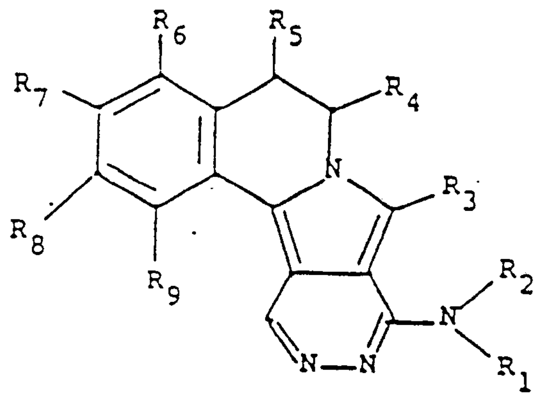

(I)

wherein

$R_1$ and $R_2$, which may be identical or different, each represent hydrogen, $C_{3-7}$ cycloalkyl, $C_{2-5}$ alkenyl, phenyl (optionally mono- or disubstituted by halogen atoms or methoxy groups), propargyl, a straight-chained or branched, saturated or unsaturated alkyl group with 1 to 5 carbon atoms which may be substituted by hydroxy, alkoxy with 1 to 4 carbon atoms, halogen, $NH_2$, NH-alkyl with 1 to 2 carbon atoms, N,N-dialkyl with 1 to 2 carbon atoms, NH-acyl with 2 to 4 carbon atoms, cycloalkyl with 3 to 7 carbon atoms, phenyl (optionally substituted by halogen, alkyl with 1 to 2 carbon atoms, alkoxy with 1 to 2 carbon atoms, NH-alkyl with 1 to 2 carbon atoms, N,N-dialkyl with 1 to 2 carbon atoms, $NH_2$, N-acyl with 2 to 3 carbon atoms), furyl, thienyl, a nitrogen-containing heterocyclic 5- or 6-membered ring which may optionally contain as a further heteroatom an oxygen or sulphur atom (and the ring may optionally be substituted by alkyl with 1 to 4 carbon atoms), or $R_1$ and $R_2$ together with the nitrogen atom represent a 3- to 7-membered ring which may optionally contain as a further heteroatom an oxygen or nitrogen atom [the ring may optionally be substituted by phenyl $(C_{1-4})$ alkyl (this phenyl ring in turn being mono- or disubstituted by halogen atoms or methoxy groups)],

or $R_2$, if $R_1$ represents hydrogen, represents $-NH_2$, di$(C_{1-2})$ alkylamino, acetonylamino, $-NH(C_{2-3})$ acyl, alkylsulphonyl or alkyloxycarbonyl group with 1 to a carbon atoms in the alkyl chain, a isoprpylideneimino group

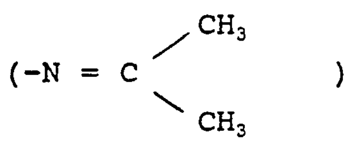

or a heterocyclic 5- or 6-membered ring containing a nitrogen atom and optionally containing as a further heteroatom an oxygen, nitrogen or sulphur atom,

$R_3$, $R_4$ and $R_5$, which may be identical or different, represent hydrogen or alkyl groups with 1 to 4 carbon atoms,

$R_7$ and $R_8$, which may be identical or different, represent hydroxy, alkoxy with 1 to 4 carbon atoms or alkylthio groups with 1 to 4 carbon atoms, and

$R_6$ and $R_9$, which may be identical or different, represent hydrogen, hydroxy, alkoxy with 1 to 4 carbon atoms or alkylthio groups with 1 to 4 carbon atoms or a group

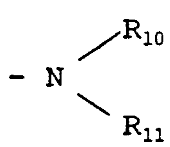

wherein

$R_{10}$ represents hydrogen or alkyl with 1 to 4 carbon atoms and

$R_{11}$ represents alkyl with 1 to 4 carbon atoms any such alkyl group being optionally substituted by a hydroxy, methoxy or furfuryl group

or a physiologically acceptable acid addition salt thereof, characterised in that a 3,4-dihydro-12-methylmercapto-pyridazino[4',5':3,4]pyrrolo[2,1-a]isoquinoline of general formula II

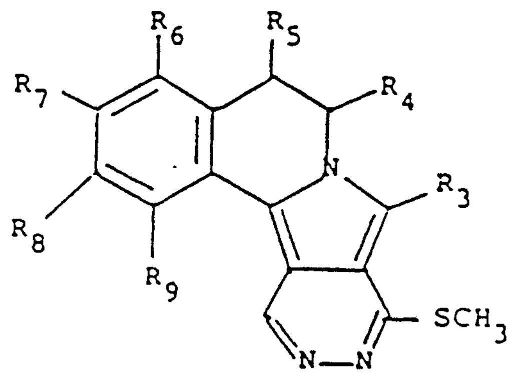

(II)

wherein
the groups $R_3$, $R_4$, $R_5$, $R_6$, $R_7$, $R_8$ and $R_9$ are as hereinbefore defined, is reacted with a compound of general formula III

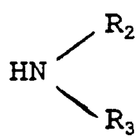

(III)

wherein
$R_1$ and $R_2$ are as hereinbefore defined, and in that a compound thus obtained is optionally converted, in a manner known per se, into a physiologically acceptable acid addition salt.

2. Process according to claim 1, characterised in that a compound of general formula I is prepared wherein $R_1$ and $R_2$, which may be identical or different, represent hydrogen or a straight-chained or branched alkyl group with 1 to 5 carbon atoms or

$R_1$ represents hydrogen and $R_2$ represents amino, methylamino, dimethylamino, isopropylideneimino, dimethylaminoalkyl with 1 to 4 carbon atoms, methoxyalkyl with 1 to 4 carbon atoms, cyclopropyl, cyclopentyl, cyclohexyl, cyclohexylmethyl, phenyl, p-halophenyl, phenylethyl, any such phenyl ring being optionally mono- or disubstituted by methoxy, or pyrazolyl,

or $R_1$ and $R_2$ together with the nitrogen atom to which they are attached represent a morpholino or piperazino ring and

$R_7$ and $R_8$ represent methoxy, or a physiologically acceptable acid addition salt thereof.

3. Process according to claim 1, characterised in that 3,4-dihydro-6,7-dimethoxy-12-dimethylamino-pyridazino[4',5':3,4]pyrrolo[2,1-a]isoquinoline or 3,4-dihydro-6,7-dimethoxy-12-isopropylaminopyridazino-[4',5':3,4]pyrrolo[2,1-a]isoquinoline

or acid addition salts thereof are prepared.

4. Use of a compound of general formula I or a physiologically acceptable acid addition salt thereof, as defined in any one of claims 1 to 3, for producing a preparation for treating cardiac insufficiency and/or cerebral metabolic disorders.

**Revendications** Pour les Etats Contractants: BE, CH, DE, FR, GB, IT, LI, LU, NL, SE

1. 12-amino-pyridazino[4',5':3,4]pyrrolo[2,1-a]isoquinoléines de formule générale I

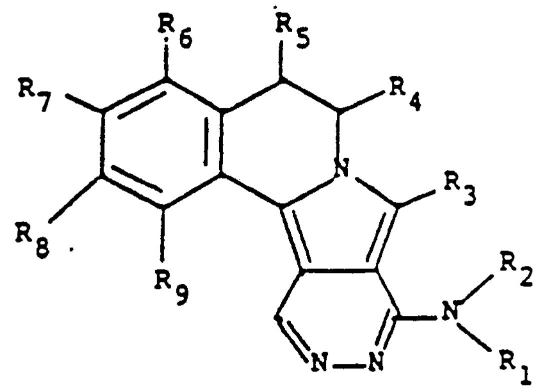

(I)

dans laquelle

$R_1$ et $R_2$, qui peuvent être identiques ou différents, représentent hydrogène, cycloalcoyle en $C_3$-$C_7$, alcényle en $C_2$-$C_5$, phényle (le cycle phényle étant éventuellement substitué une ou deux fois par halogène ou méthoxy), propargyle, un radical alcoyle, rectiligne ou ramifie, saturé ou insaturé avec 1 - 5 atomes de carbone, qui peut être substitué par hydroxy, alcoxy avec 1 - 4 atomes de carbone, halogène, $NH_2$, NH-alcoyle avec 1 - 2 atomes de carbone, N,N-dialcoyle avec 1 - 2 atomes de carbone, NH-acyle avec 2 - 4 atomes de carbone, cycloalcoyle avec 3 - 7 atomes de carbone, phényle (le cycle phényle pouvant lui-même être encore substitué par halogène, alcoyle avec 1 - 2 atomes de carbone, alcoxy avec 1 - 2 atomes de carbone, NH-alcoyle avec 1 - 2 atomes de carbone, NN-dialcoyle avec 1 - 2 atomes de carbone, $NH_2$, N-acyle avec 2 - 3 atomes de carbone), furyle, thiényle, un cycle hétérocyclique à 5 ou 6 chaînons contenant de l'azote qui peut éventuellement contenir, en tant qu'autre hétéroatome, un atome d'oxygène ou de soufre (le cycle étant éventuellement substitué par alcoyle avec 1 - 4 atomes de carbone), ou

$R_1$ et $R_2$, ensemble avec l'atome d'azote, représentent un cycle à 3 à 7 membres qui peut éventuellement contenir, en tant qu'autre hétéroatome, un atome d'oxygène ou d'azote [le cycle pouvant être éventuellement substitué par phénylalcoyle(en $C_1$-$C_4$) (le cycle phényle étant lui-méme substitué une ou deux fois par halogéne ou méthoxy)], ou

$R_2$ représente, dans le cas où $R_1$ représente l'hydrogène, un radical $-NH_2$, dialcoyl(en $C_1$-$C_2$)amino, acétonylamino, -NH-acyl(en $C_2$-$C_3$), alcoylsulfonyle ou alcoxycarbonyle avec chacun 1-3 atomes de carbone dans la chaîne alcoyle, le groupe isopropylidénimino

20 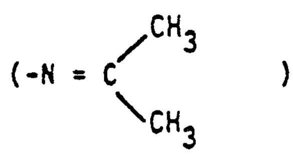

ou un hétérocycle à 5 ou 6 chaînons, contenant un atome d'azote et éventuellement, en tant qu'autre hétéroatome, un atome d'oxygène, d'azote ou de soufre,

$R_3$, $R_4$ et $R_5$, qui peuvent être identiques ou différents, représentent hydrogène ou un radical alcoyle avec 1 - 4 atomes de carbone,

$R_7$ et $R_8$, qui peuvent être identiques ou différents, représentent hydroxy, alcoxy avec 1 - 4 atomes de carbone ou alcoylthio avec 1 - 4 atomes de carbone, et

$R_6$ et $R_9$, qui peuvent être identiques ou différents, remplacent hydrogène, hydroxy, alcoxy avec 1 - 4 atomes de carbone, alcoylthio avec 1 - 4 atomes de carbone ou le radical

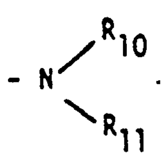

dans lequel

$R_{10}$ représente hydrogène ou alcoyle avec 1 - 4 atomes de carbone et

$R_{11}$ représente alcoyle avec 1 - 4 atomes de carbone, le radical alcoyle pouvant éventuellement être substitué par hydroxy, méthoxy ou furfuryle,

et leurs sels d'addition d'acides physiologiquement supportables.

2. Composés de formule générale I selon la revendication 1,

dans laquelle

$R_1$ et $R_2$, qui peuvent étre identiques ou différents, représentent hydrogène ou un groupe alcoyle rectiligne ou ramifié avec 1 - 5 atomes de carbone ou

$R_1$ représente hydrogène et $R_2$ représente amino, méthylamino, diméthylamino, isopropylidénimino, diméthylaminoalcoyle avec 1 - 4 atomes de carbone, méthoxyalcoyle avec 1 - 4 atomes de carbone, cyclopropyle, cyclopentyle, cyclohexyle, cyclohexylméthyle, phényle, p-halogénophényle, phényléthyle, le cycle phényle étant éventuellement substitué une ou deux fois par méthoxy, ou représente pyrazolyle,

ou $R_1$ et $R_2$, ensemble avec l'atome d'azote, représentent un cycle morpholino ou un cycle pipérazino et $R_7$ et $R_8$ représentent méthoxy,

et leurs sels d'addition d'acides physiologiquement supportables.

3. Les composés 3,4-dihydro-6,7-diméthoxy-12-diméthylamino-pyridazino[4',5':3,4]pyrrolo[2,1-a]isoquinoléine et 3,4-dihydro-6,7-diméthoxy-12-isopropylamino-pyridazino[4',5':3,4]pyrrolo[2,1-a]isoquinoléine ou leurs sels d'addition d'acides.

4. Procédé pour la préparation des composés de formule générale I selon la revendication 1 ou de leurs sels d'addition d'acides, caractérisé en ce qu'on fait réagir une 3,4-dihydro-12-méthylmercapto-pyridazino[4',5':3,4]pyrrolo[2,1-a]isoquinoléine de formule générale II

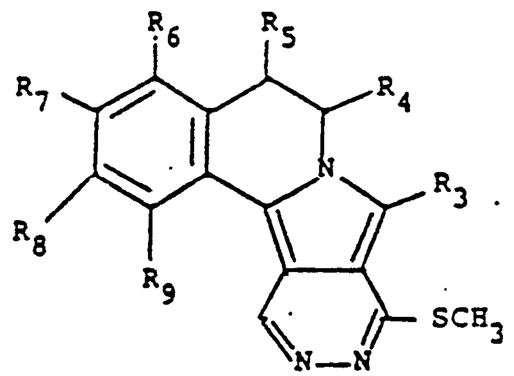

(II)

dans laquelle les radicaux $R_3$, $R_4$, $R_5$, $R_6$, $R_7$, $R_8$ et $R_9$ ont les significations indiquées plus haut, avec un composé de formule générale III

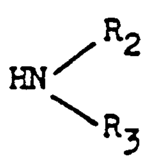

(III)

$R_1$ et $R_2$ ont les significations indiquées plus haut, et en ce qu'on transforme un composé ainsi obtenu, éventuellement de manière connue en soi, en un sel d'addition d'acide physiologiquement supportable.

5. Préparations pharmaceutiques contenant, en tant que substance active, un ou plusieurs composés de formule générale I en combinaison avec des adjuvants ou excipients usuels.

6. Procédé pour la fabrication de préparations pharmaceutiques selon la revendication 5, caractérisé en ce qu'on transforme des composés de formule générale I, avec des adjuvants et/ou excipients galéniques usuels, en formes d'utilisation pharmaceutiques usuelles.

7. Utilisation d'un composé selon l'une des revendications 1 à 3 pour la fabrication d'une préparation pour lutter contre l'insuffisance cardiaque et/ou les perturbations du métabolisme cérébral.

**Revendications** Pour l'Etat Contractant: AT

1. Procédé pour la prépration d'une 12-amino-pyridazino[4',5':3,4]pyrrolo[2,1-a]isoquinoléine de formule générale I

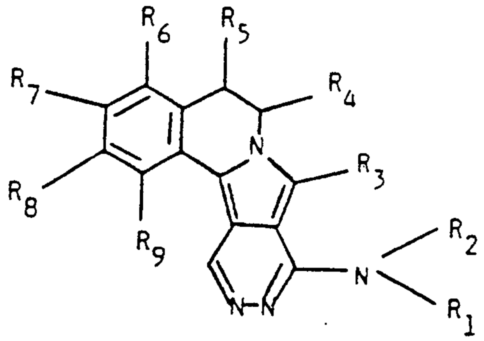

(I)

dans laquelle

$R_1$ et $R_2$, qui peuvent être identiques ou différents, représentent hydrogène, cycloalcoyle en $C_3$-$C_7$, alcényle en $C_2$-$C_5$, phényle (le cycle phényle étant éventuellement substitué une ou deux fois par halogène ou méthoxy), propargyle, un radical alcoyle, rectiligne ou ramifié, saturé ou insaturé, avec 1 - 5 atomes de carbone, qui peut être substitué par hydroxy, alcoxy avec 1 - 4 atomes de carbone, halogène, $NH_2$, NH-alcoyle avec 1 - 2 atomes de carbone, N,N-dialcoyle avec 1 - 2 atomes de carbone, NH-acyle avec 2 - 4 atomes de carbone, cycloalcoyle avec 3 - 7 atomes de carbone, phényle (le cycle phényle pouvant lui-même être encore substitué par halogène, alcoyle avec 1-2 atomes de carbone, alcoxy avec 1 - 2 atomes de carbone, NH-alcoyle avec 1 - 2 atomes de carbone, NN-dialcoyle avec 1 - 2 atomes de carbone, $NH_2$, N-acyle avec 2 - 3 atomes de carbone), furyle, thiényle, un cycle hétérocyclique à 5 ou 6 chaînons contenant de l'azote qui peut éventuellement contenir, en tant qu'autre hétéroatome, un atome d'oxygène ou de soufre (le cycle étant éventuellement substitué par alcoyle avec 1 - 4 atomes de carbone), ou

$R_1$ et $R_2$, ensemble avec l'atome d'azote, représentent un cycle à 3 à 7 membres, qui peut éventuellement contenir, en tant qu'autre hétéroatome, un atome d'oxygène ou d'azote [le cycle étant éventuellement substitué par phénylalcoyle(en $C_1$-$C_4$) (le cycle phényle étant lui-même substitué une ou deux fois par halogène ou méthoxy)], ou $R_2$ représente, lorsque $R_1$ représente hydrogène, un radical $-NH_2$, dialcoyl(en $C_1$-$C_2$)amino, acétonylamino, -NH-acyle(en $C_2$-$C_3$), alcoylsulfonyle ou alcoyloxycarbonyle avec chacun 1 - 3 atomes de carbone dans la chaine alcoyle, le groupe isopropylidénimino

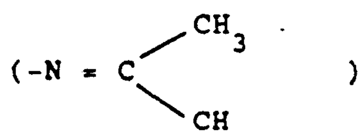

ou un cycle hétérocyclique à 5 ou 6 chaînons, contenant un atome d'azote et éventuellement, en tant qu'autre hétéroatome, un atome d'oxygène, d'azote ou de soufre,

$R_3$, $R_4$ et $R_5$, qui peuvent être identiques ou différents, représentent hydrogène ou un radical alcoyle avec 1 - 4 atomes de carbone,

$R_7$ et $R_8$, qui peuvent être identiques ou différents, représentent hydroxy, alcoxy avec 1 - 4 atomes de carbone ou alcoylthio avec 1 - 4 atomes de carbone, et

$R_6$ et $R_9$, qui peuvent être identiques ou différents, remplacent hydrogène, hydroxy, alcoxy avec 1 - 4 atomes de carbone, alcoylthio avec 1 - 4 atomes de carbone ou le radical

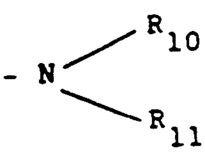

dans lequel

$R_{10}$ représente hydrogène ou alcoyle avec 1 - 4 atomes de carbone et

$R_{11}$ représente alcoyle avec 1 - 4 atomes de carbone, le radical alcoyle pouvant éventuellement être substitué par hydroxy, méthoxy ou furfuryle,

ou d'un sel d'addition d'acide physiologiquement supportable, caractérisé en ce qu'on fait réagir une 3,4-dihydro-12-méthylmercapto-pyridazino[4',5':3,4]pyrrolo[2,1-a]isoquinoléine de formule générale II

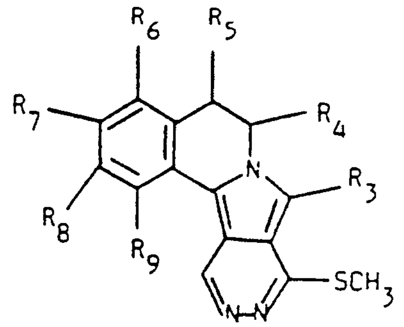

(II)

dans laquelle
les radicaux $R_3$, $R_4$, $R_5$, $R_6$, $R_7$, $R_8$ et $R_9$ ont les significations indiquées plus haut, avec un composé de formule générale III

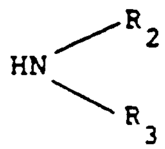

(III)

dans laquelle
$R_1$ et $R_2$ ont les significations indiquées plus haut, et en ce qu'on transforme un composé ainsi obtenu éventuellement de manière connue en soi en un sel d'addition d'acide physiologiquement supportable.

2. Procédé selon la revendication 1, caractérisé en ce qu'on prépare un composé de formule générale I, dans laquelle
$R_1$ et $R_2$, qui peuvent être identiques ou différents, représentent hydrogène ou un groupe alcoyle, rectiligne ou ramifié, avec 1 - 5 atomes de carbone ou
$R_1$ représente hydrogène et $R_2$ représente amino, méthylamino, diméthylamino, isopropylidénimino, diméthylaminoalcoyle avec 1 - 4 atomes de carbone, méthoxyalcoyle avec 1 - 4 atomes de carbone, cyclopropyle, cyclopentyle, cyclohexyle, cyclohexylméthyle, phényle, p-halogénophényle, phényléthyle, le cycle phényle pouvant être éventuellement substitué une ou deux fois par méthoxy, ou représente pyrazolyle,
ou $R_1$ et $R_2$, ensemble avec l'atome d'azote, représentent un cycle morpholino ou un cycle pipérazino et
$R_7$ et $R_8$ représentent méthoxy,
ou son sel d'addition d'acide physiologiquement supportable.

3. Procédé selon la revendication 1, caractérisé en ce qu'on prépare la 3,4-dihydro-6,7-diméthoxy-12-diméthylamino-pyridazino[4',5':3,4]pyrrolo[2,1-a]isoquinoléine ou la 3,4-dihydro-6,7-diméthoxy-12-isopropylamino-pyridazino[4',5':3,4]pyrrolo[2,1-a]isoquinoléine, ou leurs sels d'addition d'acides.

4. Utilisation d'un composé de formule générale I ou de sons sel d'addition d'acide physiologiquement supportable, défini dans l'une des revendications 1 à 3, pour la fabrication d'une préparation pour lutter contre l'insuffisance cardiaque et/ou les perturbations du métabolisme cérébral.